# EUROPEAN PATENT APPLICATION

(11) **EP 2 633 909 A2**
(43) Date of publication of application: **04.09.2013**
(21) Application number: 12175835.3
(22) Date of filing: 11.07.2012
(51) Int. Cl.: B01L 3/00, B01L 7/00

(54) **Bio-reaction device chip**

(30) Priority: 29.02.2012 KR 20120021265
(71) Applicant: K-Mac, Daejeon 305-500 (KR)
(72) Inventor: Seo, Sung-Min, Seoul 122-930 (KR); Park, Jae-Hyung, Daejeon 306-804 (KR); Oleksandrov, Sergiy, Daejeon 305-503 (KR); Lee, Joong Hwan, Daejeon 306-110 (KR); Paek, Mun-Cheol, Daejeon 305-755 (KR); Ku, Su-Jin, Daejeon 305-761 (KR)
(74) Representative: Stolmár & Partner

(57) **Abstract**

Provided is a bio-reaction device chip for confirming or detecting a biomaterial reaction, and more particularly, is a bio-reaction device chip, where a plurality of wells are formed in the plate to contain biomaterials to be tested, thereby simultaneously analyzing various targets, and the well is formed to have a structure of two or more steps such that the sample to be tested is contained in the lower well and a protective layer for protecting the sample, such as oil, is contained in the upper well, thereby preventing evaporation of the sample due to heating at the time of the biomaterial reaction, and thus, improving reliability in analysis.

## Description

### CROSS-REFERENCE TO RELATED APPLICATIONS

This application claims priority under 35 U.S.C. §119 to Korean Patent Application No. 10-2012-0021265, filed on 02,29,2012 in the Korean Intellectual Property Office, the disclosure of which is incorporated herein by reference in its entirety.

### TECHNICAL FIELD

The following disclosure relates to a bio-reaction device chip for confirming or detecting a biomaterial reaction, and more particularly to a bio-reaction device chip capable of simultaneously analyzing various targets by having a plurality of wells formed in a plate to contain a biomaterial to be tested, and improving reliability in analysis by having a structure where evaporation of the biomaterials due to the reaction can be prevented.

### BACKGROUND

Generally, a DNA microarray (DNA chip) technology is used in order to confirm or detect genes, infection sources, germs, mutants, genotyping, gene expression, or the like.

This technology is realized by immobilizing a nucleic acid probe or the like on various solid surfaces such as a glass substrate, a metal substrate, a bead, a plastic, and the like, and has been employed for diagnosis in hospitals or clinics since many different targets can be simultaneously analyzed.

Meanwhile, convenience and reliability are very important factors in this clinical diagnosis. In cases of the existing DNA microarrays and protein chips, a sticker enabling the formation of a chamber called a frame seal is attached on a flat slide glass, and a solution is put thereinto, so that a bio-reaction is allowed to proceed. However, since the seal needs to be torn and washed after the reaction, this work may require a large amount of labor and skilled workers and moreover be cumbersome. That is, a procedure of injecting the amplified gene through an inlet of the chamber consisting of the frame seals is cumbersome; if air is infiltrated during the injection procedure, bubbles are formed, which fails to induce the reaction; attached frame seals need to be torn, and then immersed and washed in a washing buffer; and when a lid of a tube containing the amplified gene is opened in order to inject the amplified gene into a DNA chip, aerosol contamination occurs from this moment.

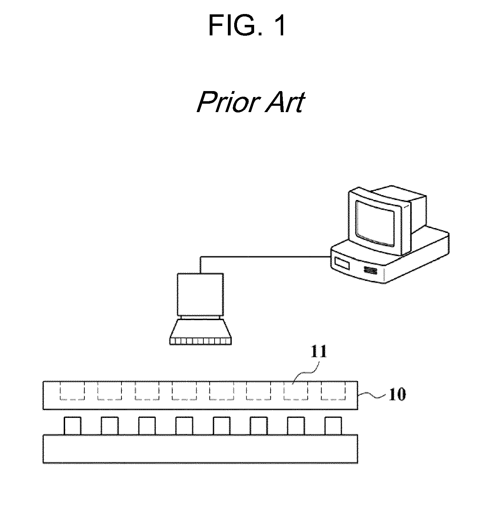

In addition, a multi-well plate where a plurality of wells (containing spaces) are formed in a plate shape such that samples are contained therein for reaction with biomaterials is sometimes used. As shown in FIG. 1, the plurality of wells are formed in an upper surface of a flat plate shaped plate to contain samples, and thus, various kinds of samples may be tested and analyzed at one time. However, since the wells are formed in a single groove type, the samples may be evaporated at the time of heating for a reaction with the sample or other materials may be inputted thereinto, which causes reliability in analysis to be deteriorated.

In this regard, Korean Laid-Open Publication No. 10-2009-0044144 discloses a microplate sample analysis apparatus.

### [Related Art Documents]

### [Patent Document]

### (Patent Document 1) KR 10-2009-0044144 A (2009.05.07.)

### SUMMARY

An embodiment of the present invention is directed to providing a bio-reaction device chip, capable of simultaneously analyzing various targets by having a plurality of wells formed in a plate so as to contain biomaterials to be tested, and capable of improving reliability in analysis by having a structure where evaporation of the biomaterials due to a biomaterial reaction can be prevented.

In one general aspect, there is provided a bio-reaction device chip, including: a plate 100; and a plurality of wells 200 concavely formed in one surface of the plate 100, wherein the well 200 includes an upper well 210 formed at an upper portion thereof and a lower well 220 formed below the upper well 210 and having a smaller diameter than the upper well 210.

The bio-reaction device chip may further include a detector 300 formed by coating or arraying (arranging) materials selected from protein, nucleic acid, peptide nucleic acid (PNA), aptamer, or antibody, on a bottom surface of the lower well 220.

The well 200 may have two or more steps where a lower step has a smaller diameter than an upper step.

### BRIEF DESCRIPTION OF THE DRAWINGS

FIG. 1 is a schematic view showing a micro-plate sample analysis apparatus of the related art;

FIG. 2 is a perspective view showing a bio-reaction device chip of the present invention;

FIG. 3 is an upper plane view showing the bio-reaction device chip of the present invention;

FIG. 4 is a cross-sectional view showing the bio-reaction device chip of the present invention; and

FIG. 5 and 6 are a cross-sectional view showing a structure where a sample and an oil are contained in a well according to the present invention.

### [Detailed Description of Main Elements]

1000: bio-reaction device chip (of the present invention)
100: plate
200: well
210: upper well 220: lower well
300: detector
400: sample
500: oil

### DETAILED DESCRIPTION OF EMBODIMENTS

Hereinafter, a bio-reaction device chip of the present invention will be described in detail with reference to the accompanying drawings.

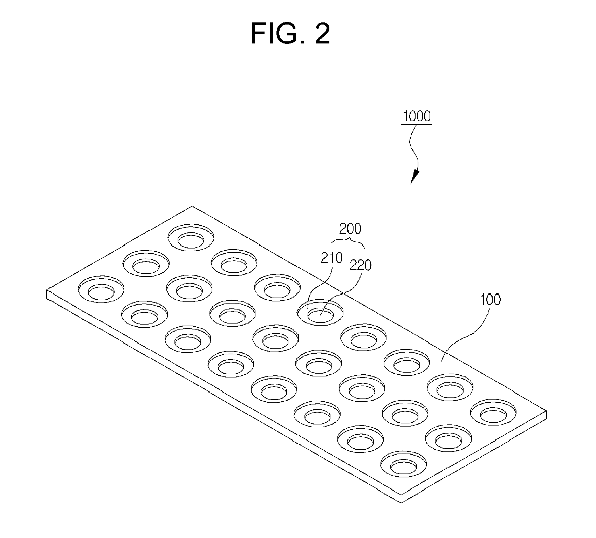

FIG. 2 is a perspective view showing a bio-reaction device chip of the present invention.

As shown in the drawings, a bio-reaction device chip 1000 of the present invention includes a plate 100; and a plurality of wells 200 concavely formed in one surface of the plate 100. The well 200 includes an upper well 210 formed at an upper portion thereof and a lower well 220 formed below the upper well 210. The lower well has a smaller diameter than the upper well 210.

First, the plate 100 may be formed in a flat plate type, and may be formed of various materials such as a glass substrate, a metal plate, a plastic plate, and the like.

The well 200 may be formed in a concave shape by performing etching or physical processing on a surface of the plate 100. Here, the well 200 may be formed to have multiple steps, and an embodiment of the present invention where the well 200 has two steps will be described.

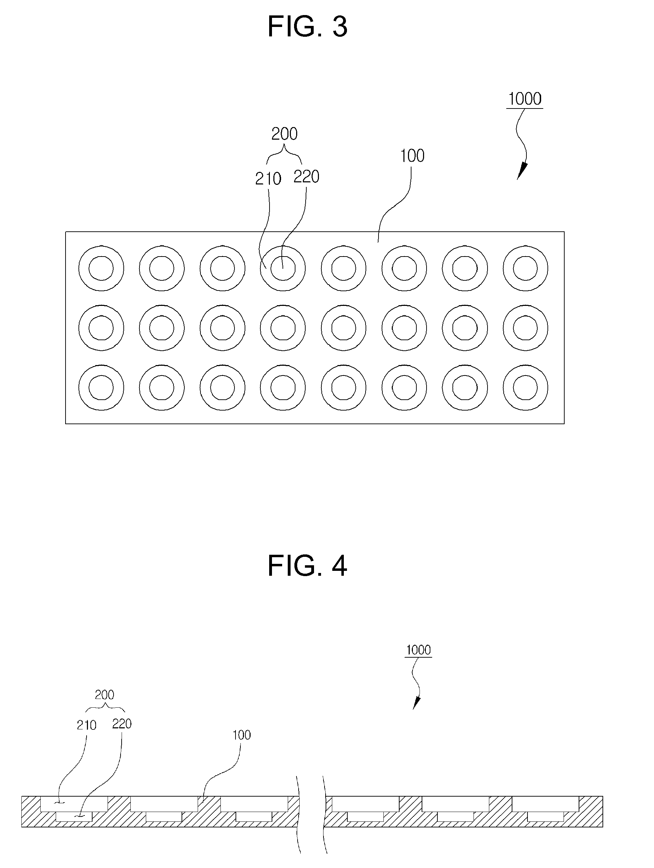

As shown in FIGS. 3 and 4, with respect to the well 200, the upper well 210 is formed in the upper surface of the plate 100 and the lower well 220 is formed below the upper well 210 while the upper well 220 has a large diameter and the lower well 220 has a smaller diameter than the upper well 210.

Here, the upper well 210 and the lower well 220 may be formed to have various diameters and heights. In the present invention, the well is formed in a micro unit, such that a sample for the biomaterial reaction and an oil for protecting the sample above the sample are contained.

Here, as for the method of forming the upper well 210 and the lower well 220, first, a mask pattern of resist or the like is formed on the upper surface of the plate 100 such that a portion thereof corresponding to the diameter of the upper well 210 is emptied, and then etching is performed, to thereby form the upper well 210 having a particular depth. In addition, after the upper well 210 is formed, a doughnut shape of mask pattern is again formed on a bottom surface of the upper well 210 such that a portion thereof corresponding to the diameter of the lower well 220 is emptied, and then etching is again performed, to thereby form the lower well 220 having a particular depth. In addition, the above mask patterns may be removed and washed, to thereby form a well structure having a desired shape (in two steps).

Another method, the well 200 may be formed through physical processing, and the well 200 may be formed to have two steps as described above, by using various methods, such as, cut processing, laser processing, discharge processing, or the like, depending on the material of the plate 100.

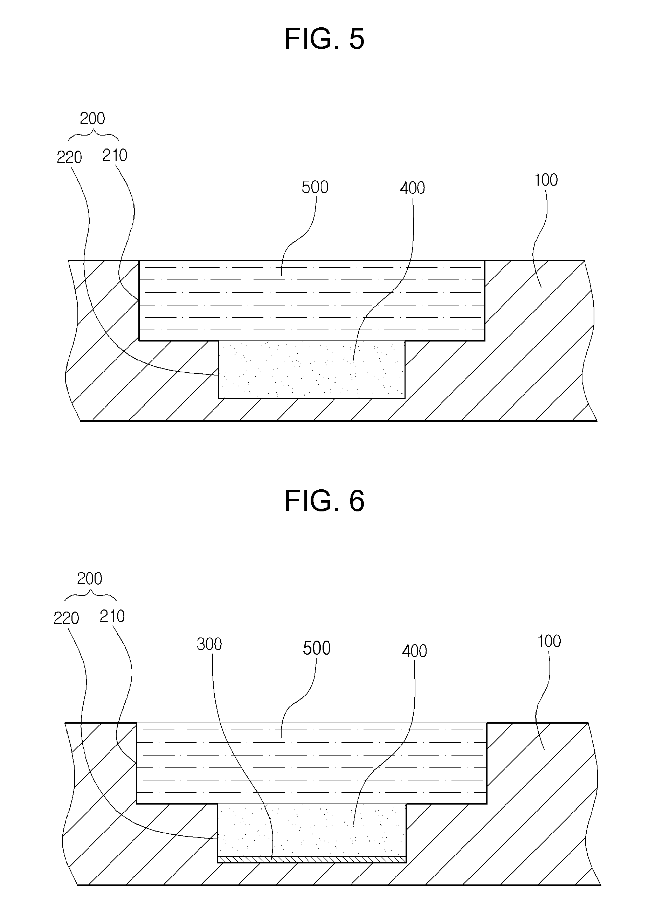

The bio-reaction device chip of the present invention constituted as above is used in a gene amplification reaction and the like, and for the use thereof, a sample 400 to be tested is injected in the lower well 220 and an oil 500 for protecting the sample 400 is injected in the upper well 210 above the lower well 220, as shown in FIG. 5. Hence, a layer of the oil 500 injected in the upper well 210 is covering the sample 400 contained in the lower well 220. Here, in the case where the well is formed to have one step, the sample is injected thereinto by a predetermined height and the oil is injected thereon, so that an upper surface of the sample is covered with the oil layer. Hence, the oil and the sample may be leaned to one side, or may be mixed with each other. Even though they are not mixed with each other at the time of injection, the heating for reaction of the sample may cause the oil and the sample to lean toward one side or mixed with each other, thereby deteriorating the reliability of the biomaterial reaction. Whereas, in the present invention, the well is formed to have two steps, so that the oil covers the sample, but the sample and the oil is not mixed with each other due to the physical structure thereof, and thus, evaporation of the sample due to heating of the sample can be prevented. Also, since the well is formed to have two steps, the injection amount of sample and the injection amount of oil can be uniformly controlled. In addition, the sample is covered with the oil, to thereby prevent external impurities from infiltrating to the sample, and thus, reliability in analysis can be improved.

As described above, according to the bio-reaction device chip of the present invention, a plurality of wells are formed in the plate to contain biomaterials to be tested, thereby simultaneously analyzing various targets, and the well is formed to have a structure of two or more steps such that the sample to be tested is contained in the lower well and a protective layer for protecting the sample, such as oil, is contained in the upper well, and thus, evaporation of the sample due to heating at the time of the biomaterial reaction can be prevented, thereby improving reliability in analysis.

In addition, the procedure of injecting the amplified gene into the well is simplified; air is never infiltrated to the well during the injection procedure; and the there is no cumbersomeness in tearing the frame seals from the existing DNA chip at the time of washing since the sample in the well is simply removed and immersed in the washing buffer at the time of washing.

Further, the sample in the well is automatically washed in the same principle as an ELISA automated washer, and thus, the cumbersomeness of tearing and washing the frame seals from the existing DNA chip can be eliminated due to the automation.

Meanwhile, when the cap of the tube containing the amplified gene is opened in order to inject the amplified gene to the DNA chip, aerosol contamination occurs from this moment. Therefore, in the present invention, a separate process of transferring the amplified gene to the DNA chip is skipped and gene amplification and hybridization can be carried out directly on the DNA chip. Hence, the gene amplification proceeds on the DNA chip, and subsequently hybridization directly proceeds in the same state, and thus, the process cumbersomeness of transferring the amplified gene and aerial contamination caused by this process can be eliminated.

In addition, in the bio-reaction device chip 1000 of the present invention, a detector 300 may be formed by coating or arraying (arranging) materials selected from protein, nucleic acid, peptide nucleic acid (PNA), aptamer, or antibody, on a bottom surface of the lower well 220, as shown in FIG. 6. That is, the detector 300 capable of detecting a biomaterial is formed on the bottom surface of the lower well 220, like the DNA chip; the sample 400 is injected to the lower well 220; the oil is injected to the upper well 210; and then gene amplification and hybridization procedures proceed, so that the amplified gene can be conjugated with and reacted with the target gene probes of the detector 300. Hence, a fluorescent signal generated during this procedure may be obtained by using a monitoring apparatus.

The bio-reaction device chip 1000 of the present invention may be formed to have multiple steps such as two or more steps. The well 200 may be formed to have two or more steps while the lower step has a smaller diameter than the upper step.

That is, as such, the well 200 may be formed to have two steps, or multiple steps such as two or more steps. In the case where the well 200 is formed to have three steps, first-, second-, and third-step wells are formed from the upper surface of the plate 100 in the downward direction. A sample is contained in the third-step well which is the lowest, an oil for protecting the sample is contained in the second-step well above the first-step well, and a protecting cap or the like is combined in the first-step well above the second-step well.

As described above, the bio-reaction device chip of the present invention is utilized, to thereby perform real-time gene amplification during molecular diagnosis. For example, the real-time gene amplification can be performed on the chip, by injecting a solution for the biomaterial reaction the well; covering the sample with the oil in order to prevent evaporation of the sample during the gene amplification; carrying out the gene amplification; and obtaining a fluorescent signal generated in the micro-well.

As such, the biomaterial detecting device of the present invention may be variously applied in various experiment fields below due to the above advantages.

(1) DNA chip analysis: A plurality of target gene probes (detectors) are arrayed and immobilized on a bottom of the lower well of the multiple wells. The sample obtained by carrying out a gene amplification reaction outside is transferred to the well; and then, a hybridization reaction is carried out.

(2) Gene amplification + DNA chip analysis: A plurality of target gene probes (detectors) are arrayed and immobilized on a bottom of the lower well of the multiple wells; a reagent for gene amplification is inputted to the lower well; the sample is covered with an oil in the upper well, in order to prevent evaporation of the sample during the gene amplification procedure, and then gene amplification followed by a hybridization reaction are carried out.

(3) Real-time PCR + DNA chip analysis: The bio-reaction device chip of the present invention having multiple-step wells may be utilized as a chip for PCR. That is, a reagent for gene amplification for performing real-time PCR is injected to the lower well; the sample is covered with the oil in the upper well in order to prevent evaporation of the sample during the gene amplification procedure; and a fluorescent signal generated while the gene amplification is carried out under the proceeding of a temperature cycle is obtained in real-time by using a camera, and thus, the real-time PCR can be realized.

(4) Real-time PCR + DNA chip analysis: A plurality of target gene probes are arrayed and immobilized on a bottom of the lower well; the real-time PCR of Item 3) is carried out to thereby in real-time obtain a fluorescent signal generated in the well during the gene amplification procedure by using a camera; a hybridization reaction is carried out, followed by washing; and a fluorescent signal reacting with the probes (detectors) immobilized on the bottom surface of the lower well is obtained, and thus, DNA chip analysis is carried out. Therefore, the real-time PCR and the DNA chip analysis can be integratedly performed.

(5) ELISA analysis: The bio-reaction device chip of the present invention having multiple-step wells may serve as the existing plastic micro-titer plate, and thus, may be also utilized in immunity analysis. An antibody to be immobilized is injected to the lower well, to thereby be coated and immobilized on the entire bottom of the lower well, followed by washing; a residual portion is filled with a blocking reagent, followed by washing; a sample to be tested is injected to the lower well or the upper well, to thereby induce an antigen-antibody reaction, followed by washing; in order to confirm the presence or absence of the antibody-antigen reaction, a secondary antibody for signal confirmation is again injected, to thereby induce the reaction, followed by washing; and a fluorescent or a substrate is injected to obtain a signal of color formation or light emission.

(6) RIA analysis: Reactivity may be confirmed by carrying out the same procedure as Item 4) to obtain a radioisotope signal as a final signal.

(7) Protein array (Protein chip): A plurality of target antibodies are arrayed and immobilized on the bottom surface of the lower well, and the same procedure as Item 4), from the process of blocking the residual portion, is carried out to obtain a signal.

As set forth above, according to the bio-reaction device chip of the present invention, a plurality of wells are formed in the plate to contain biomaterials to be tested, thereby simultaneously analyzing various targets, and the well is formed to have a structure of two or more steps such that the sample to be tested is contained in the lower well and a protective layer for protecting the sample, such as oil, is contained in the upper well, and thus, evaporation of the sample due to heating at the time of the biomaterial reaction can be prevented, thereby improving reliability in analysis.

The present invention is not limited to the above-mentioned embodiments and an applied range thereof may be various. Also, various modifications of the present invention may be made by those skilled in the art without departing from the gist of the present invention.

## Claims

1. A bio-reaction device chip, comprising:
a plate 100; and
a plurality of wells 200 concavely formed in one surface of the plate 100,
wherein the well 200 includes an upper well 210 formed at an upper portion thereof and a lower well 220 formed below the upper well 210 and having a smaller diameter than the upper well 210.

2. The bio-reaction device chip of claim 1, further comprising a detector 300 formed by coating or arraying (arranging) materials selected from protein, nucleic acid, peptide nucleic acid (PNA), aptamer, or antibody, on a bottom surface of the lower well 220.

3. The bio-reaction device chip of claim 1, wherein the well 200 has two or more steps where a lower step has a smaller diameter than an upper step.
